(19) Europäisches Patentamt
European Patent Office
Office européen des brevets

(11) Numéro de publication: **0 514 277 B1**

(12) **FASCICULE DE BREVET EUROPEEN**

(45) Date de publication de fascicule du brevet: **14.12.94**

(51) Int. Cl.5: **C07D 237/20**, A61K 31/50, C07D 451/02, C07D 451/04, C07D 487/04

(21) Numéro de dépôt: **92401333.7**

(22) Date de dépôt: **15.05.92**

(54) **Dérivés de 3-aminopyridazines actifs sur le système nerveux central.**

(30) Priorité: **16.05.91 FR 9105973**

(43) Date de publication de la demande:
**19.11.92 Bulletin 92/47**

(45) Mention de la délivrance du brevet:
**14.12.94 Bulletin 94/50**

(84) Etats contractants désignés:
**AT BE CH DE DK ES FR GB GR IT LI LU NL PT SE**

(56) Documents cités:
**EP-A- 0 072 726**
**EP-A- 0 382 634**
**EP-A- 0 382 635**
**EP-A- 0 429 344**

**JOURNAL OF MEDICINAL CHEMISTRY. vol. 32, no. 3, Mars 1989, WASHINGTON US pages 528- 537; C. WERMUTH: '3-aminopyridazine derivatives with atypical antidepressant, serotonergic and dopaminergic activities'**

(73) Titulaire: **SANOFI**
**32-34, rue Marbeuf**
**F-75008 Paris (FR)**

(72) Inventeur: **Bourguignon, Jean-Jacques**
**14, Rue du Bruhly**
**F-67150 Hipsheim (FR)**
Inventeur: **Wermuth, Camille Georges**
**3, Rue de la Côte d'Azur**
**F-67100 Strasbourg (FR)**
Inventeur: **Kan, Jean-Paul**
**7, Rue du Vallon**
**F-34830 Clapiers (FR)**

(74) Mandataire: **Gillard, Marie-Louise et al**
**Cabinet Beau de Loménie**
**158, rue de l'Université**
**F-75340 Paris Cédex 07 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne des dérivés de 3-aminopyridazines actifs sur le système nerveux central.

De nombreux dérivés pyridaziniques ont été proposés comme médicaments, notamment actifs sur le système cardiovasculaire ou sur le système nerveux central.

Plus particulièrement, le brevet FR-A-2510998 et le brevet EP-A-72726 décrivent des pyridazines diversement substituées et portant toutes en position 3 un substituant aminé du type

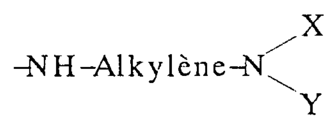

dans lequel X et Y représentent indépendamment l'hydrogène, un groupe alkyle ou forment avec l'atome d'azote auquel ils sont liés un hétérocycle tel que la morpholine. Ces composés possèdent une activité sur le système nerveux central en tant qu'antidépresseurs.

Les demandes de brevet européen EP-A-0 382 634 et EP-A-0 382 635, décrivent des dérivés de pyridazine diversement substitués sur le cycle pyridazinique et portant en position 3 un substituant aminé du type.

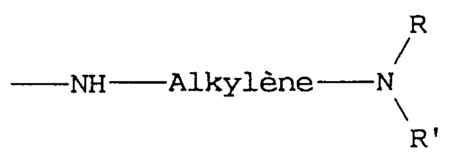

dans lequel le groupe alkylène est linéaire et un des groupes R et R' peut éventuellement représenter l'hydrogène.

Ces composés présentent une activité en tant que ligands des récepteurs cholinergiques.

Selon la présente invention, on a maintenant trouvé de nouveaux dérivés de 3-aminopyridazines dépourvus d'activité antidépressive et possédant une activité en tant que ligands des récepteurs cholinergiques, plus particulièrement comme ligands des récepteurs muscariniques de type $M_1$.

La présente invention concerne l'obtention de pyridazines de formule :

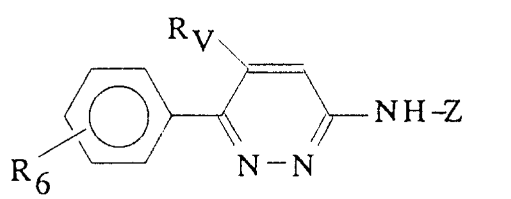

(I)

dans laquelle :

- $R_V$ représente un groupe alkyle droit ou ramifié en $C_1$-$C_4$ ;
- $R_6$ représente l'hydrogène, un alcoxy en $C_1$-$C_3$ ou un groupe hydroxyle;
- Z représente un groupe :

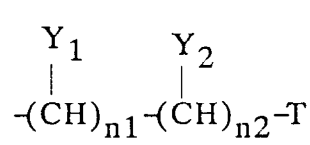

dans lequel :

. $n_1$ et $n_2$ représentent indépendamment zéro ou un,

. $Y_1$ et $Y_2$ représentent chacun l'hydrogène,

. T représente un hétérocycle choisi parmi :

a)

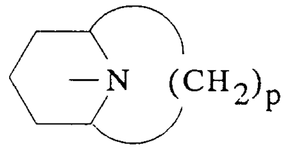

lorsque $n_1 = n_2 = 1$
et p représente alors 2 ou 3 ;

b)

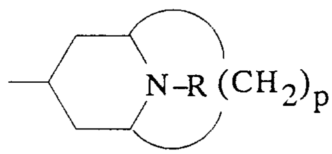

lorsque $n_1 = n_2 = 0$
et p représente alors 2 ou 3, R = alkyle $C_1$-$C_3$ ;

c)

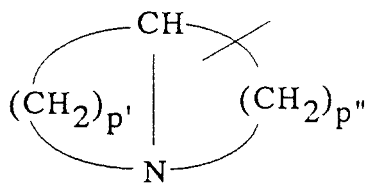

lorsque $n_1 = 1$, $n_2 = 0$
et p' et p'' représentent alors 3 ou 4 ;

ainsi que leurs sels avec des acides organiques ou minéraux.

Les composés préférés de l'invention sont deux dans lesquels :

. soit $n_1$ et $n_2$ sont égaux à un et T représente l'hétérocycle a) dans lequel p est égal à 2, à savoir le groupe 8-azabicyclo[3.2.1]oct-8-yl;
. soit $n_1$ et $n_2$ sont égaux à zéro et T représente l'hétérocycle b) dans lequel p est égal à 2 et R représente un groupe éthyle, à savoir le groupe 8-éthyl-8-azabicyclo[3.2.1]oct-3-yl;
. soit $n_1$ est égal à un et $n_2$ est égal à zéro et T représente l'hétérocycle c) dans lequel p' = p'' = 3, à savoir le groupe 1-azabicyclo[3.3.0]oct-5-yl;

ainsi que leurs sels avec des acides minéraux ou organiques.

Les sels des composés de formule (I) selon la présente invention comprennent aussi bien ceux avec des acides minéraux ou organiques qui permettent une séparation ou une cristallisation convenable des composés de formule (I), tels que l'acide picrique ou l'acide oxalique, que ceux qui forment des sels pharmaceutiquement acceptables tels que le chlorhydrate, le bromhydrate, le sulfate, l'hydrogénosulfate, le dihydrogénophosphate, le méthanesulfonate, le maléate, le fumarate, le 2-naphtalène-sulfonate.

Selon un autre de ses aspects, la présente invention concerne un procédé pour la préparation des composés de formule (I).

Ce procédé est caractérisé en ce que l'on fait réagir une amine de formule :

$H_2N$-Z (II)

dans laquelle Z est tel que défini précédemment pour (I), sur la 3-chloropyridazine de formule :

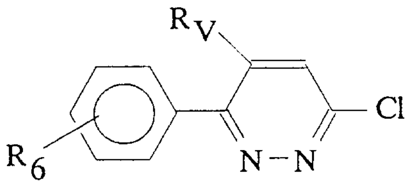

(III)

dans laquelle $R_V$ et $R_6$ sont tels que définis précédemment pour (I), et éventuellement, on salifie le composé ainsi obtenu avec un acide minéral ou organique.

La réaction de substitution de la 6-chloropyridazine (III) par l'amine (II) est réalisée à une température comprise entre 100 et 150 ° C, sans solvant ou en présence d'un solvant inerte comme un alcanol et éventuellement en présence de chlorure d'ammonium. Le composé (I) est alors isolé et purifié selon les méthodes habituelles. Le produit ainsi obtenu est isolé sous forme de base libre ou de sel selon les techniques conventionnelles.

Lorsque le composé de formule (I) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le 2-naphtalènesulfonate.

A la fin de la réaction, le composé de formule (I) peut être isolé sous forme d'un de ses sels, par exemple le chlorhydrate ; dans ce cas, s'il est nécessaire, la base libre peut être préparée par neutralisation dudit sel avec une base minérale ou organique telle que l'hydroxyde de sodium ou la triéthylamine ou avec un carbonate ou bicarbonate alcalin, tel que le carbonate ou bicarbonate de sodium ou de potassium.

Lorsque les composés (I) possèdent un centre chiral et sont obtenus sous forme de mélange racémique, les énantiomères correspondants, qui peuvent être séparés selon des méthodes classiques, font partie de l'invention.

Les 6-chloropyridazines (III), utilisées comme produit de départ, sont préparées à partie des 2*H*-pyridazin-3-ones de formule (IV):

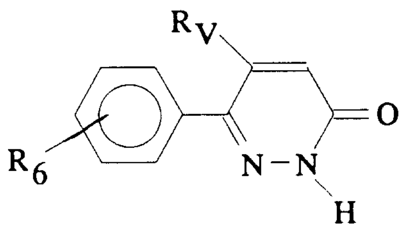

(IV)

par action d'un excès d'oxychlorure de phosphore, à chaud, sans solvant ou en présence d'un solvant inerte tel que par exemple l'acétonitrile.

Les 2*H*-pyridazin-3-ones (IV) sont connues ou préparées selon des méthodes connues selon le schéma réactionnel suivant :

## SCHEMA 1

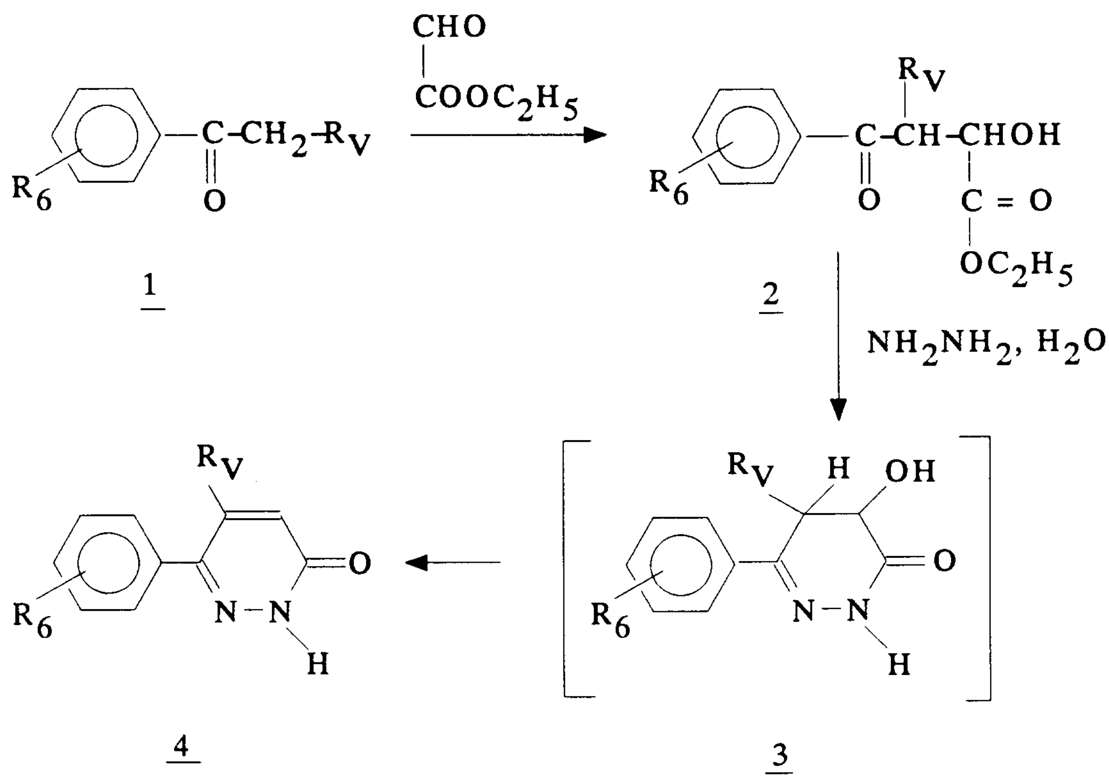


1 2 3 4

La réaction d'aldolisation effectuée entre un dérivé 1 de l'acétophénone et le glyoxylate d'éthyle permet la préparation de l'hydroxycétoester 2 ; celui-ci est alors cyclisé avec l'hydrate d'hydrazine pour fournir le composé 3 qui n'est pas isolé.

Le passage des hydroxycétoesters 2 aux pyridazones 4 est effectué en une seule étape qui réunit la cyclisation et la déshydratation.

Les amines $H_2N$-Z (II) sont connues ou ont été préparées selon des méthodes connues.

Les schémas réactionnels ci-dessous illustrent de façon non limitative la préparation des amines $H_2N$-Z (II).

Lorsque $n_1 = n_2 = 1$, et T est l'hétérocyle a) dans lequel $p = 2$ ou 3, l'amine correspondante (II) est préparée selon le schéma réactionnel suivant :

SCHEMA 2

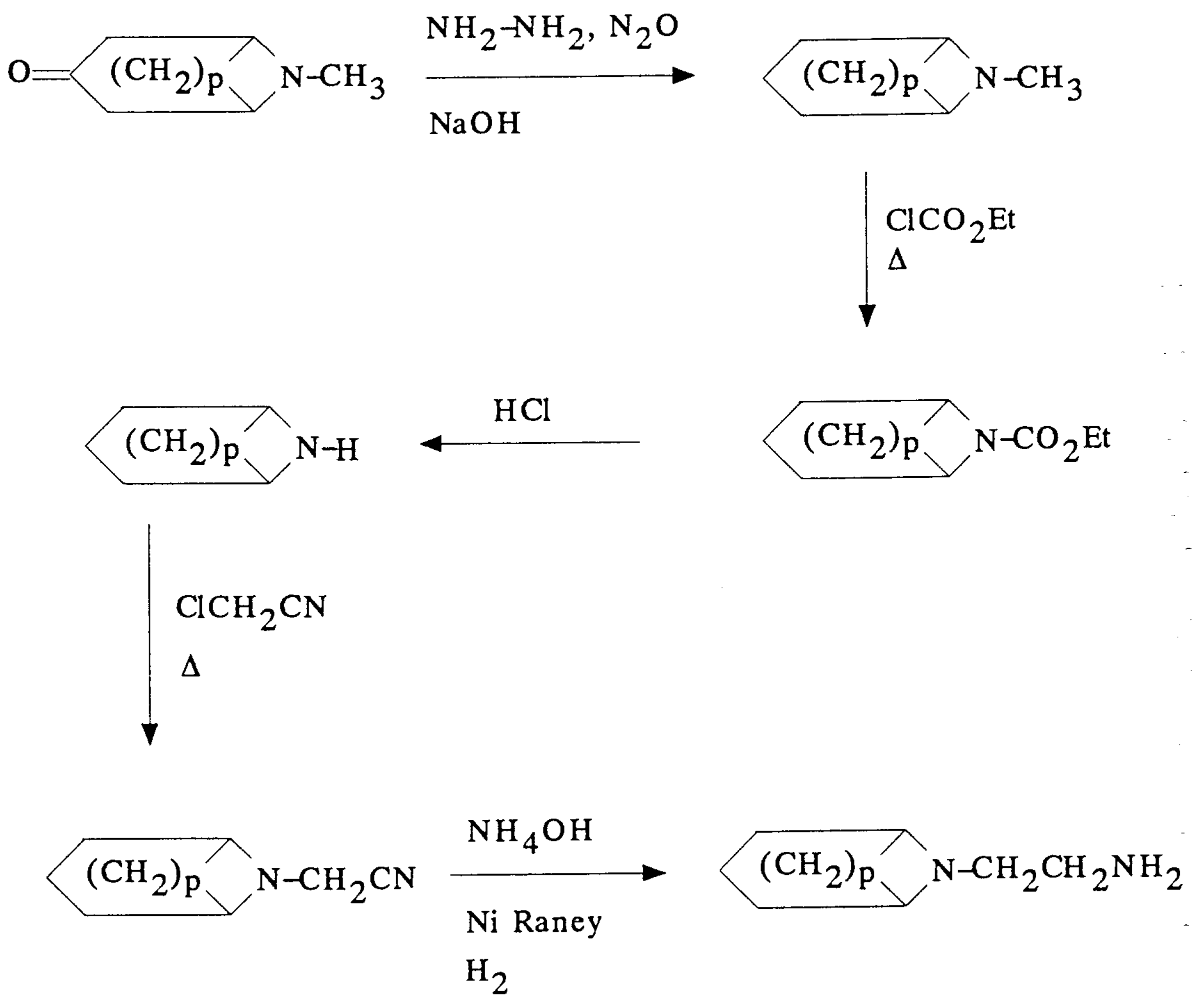


Lorsque $n_1 = n_2 = 0$ et lorsque T représente l'hétérocycle b), l'amine correspondante (II) est préparée selon la méthode décrite par Dostert et al., Eur. J. Med. Chem. - Chim. Ther., 1984, 19 (2), 105-110 selon le schéma 3 suivant qui permet la préparation des composés (II) dont le groupe $-NH_2$ est soit en configuration équatoriale (IIe) soit en configuration axiale (IIa).

## SCHEMA 3

- Configuration du $-NH_2$ : équatoriale

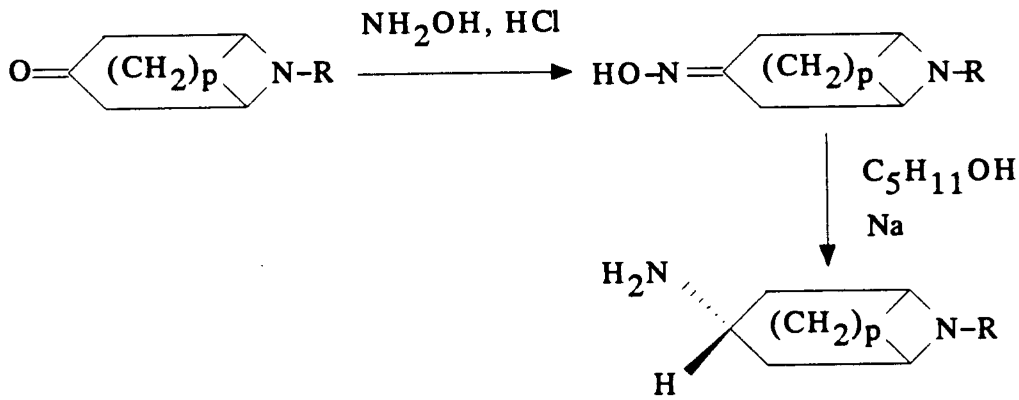


(IIe)

- Configuration du $-NH_2$ : axiale

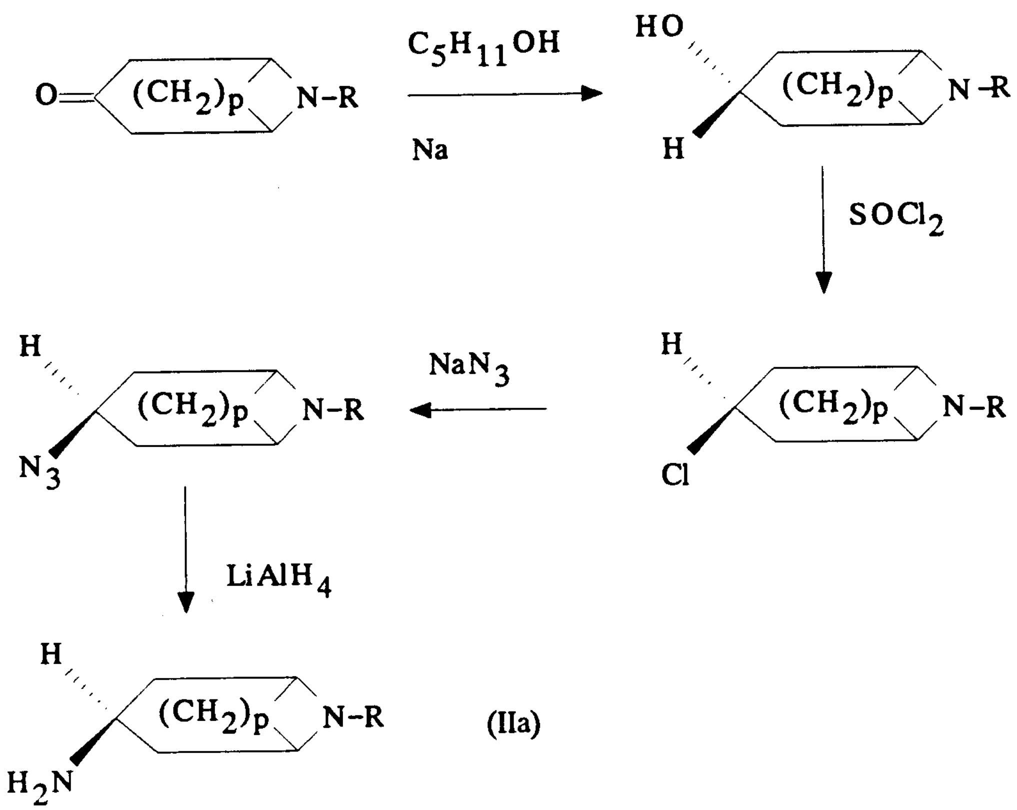


(IIa)

Lorsque $n_1$ = 1, $n_2$ = 0 et T représente le groupe 1-azabicyclo[3.3.0]oct-5-yl (hétérocycle c), p' = p'' = 3), l'amine correspondante 1-azabicyclo[3.3.0]oct-5-yl-méthylamine est préparée selon Miyano et al., J. Heterocyclic Chem., 1982, 19, 1465 ; Synthesis, 1978, 701; Miyano et al., J. Heterocyclic Chem., 1987, 24, 47 selon le schéma réactionnel suivant :

SCHEMA 4

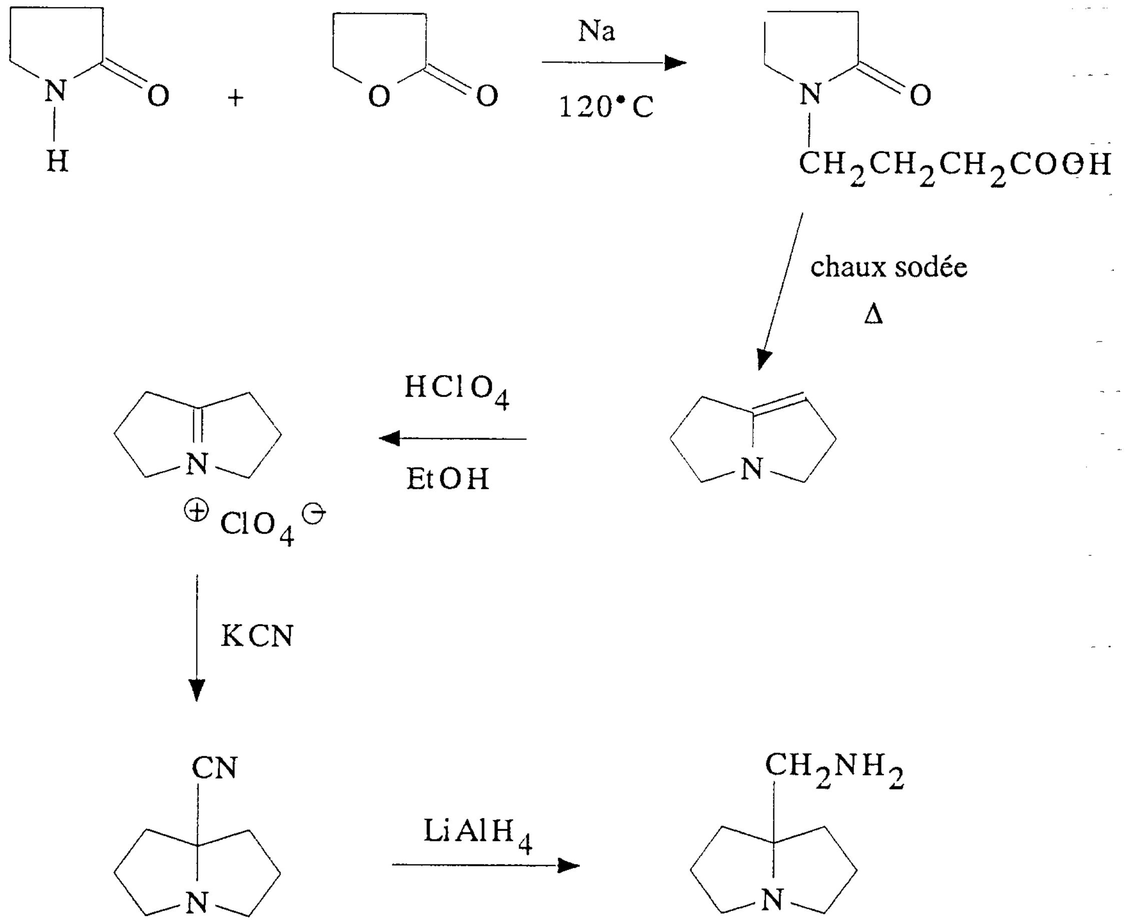


L'hétérocycle c) peut également être préparé selon J. Med. Chem., 1985, 28, 714 ou selon le brevet EP-287356.

Les exemples suivants illustrent l'invention sans toutefois la limiter.

Les points de fusion, F, ont été mesurés au banc chauffant Koffler.

EXEMPLE 1

3-[2-(8-azabicyclo[3.2.1]oct-8-yl)éthylamino]-6-(2-hydroxyphényl)-5-méthylpyridazine.

$R_6$ = 2-OH; $R_V$ = $-CH_3$ ;
$n_1$ = $n_2$ = 1 ;

T =

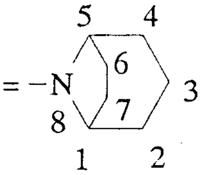

A) 3-[2-(8-azabicyclo[3.2.1]oct-8-yl)éthylamino]-6-(2-méthoxyphényl)-5-méthylpyridazine.

1,04 g de 2-(8-azabicylo[3.2.1]oct-8-yl)éthylamine, 1,58 g de 3-chloro-5-méthyl-6-(2-méthoxyphényl)-pyridazine et 0,38 g de chlorure d'ammonium sont mis en solution dans 2,5 ml de butanol et chauffés à reflux pendant 48 heures sous argon. Le mélange réactionnel est concentré sous vide puis on ajoute au résidu une solution aqueuse de carbonate de potassium à 20 % jusqu'à pH = 13. Le mélange est extrait avec de l'acétate d'éthyle, la phase organique est séparée par décantation et on y ajoute alors 30 ml d'une solution aqueuse d'acide citrique à 10 %. La phase aqueuse est séparée, lavée à l'acétate d'éthyle et alcalinisée avec 30 ml d'une solution aqueuse d'hydroxyde de sodium à 33 %. L'huile formée est extraite avec de l'acétate d'éthyle, la phase organique est séparée, séchée sur $Na_2SO_4$, filtrée et concentrée sous vide.

Le résidu est purifié par chromatographie sur alumine, éluant : acétate d'éthyle puis acétate d'éthyle-méthanol 9-1 (v/v) additionné de 2 % de triéthylamine.

La concentration des fractions de produit pur fournit 1,2 g du produit attendu.

B) 3-[2-(8-azabicyclo[3.2.1]oct-8-yl)éthylamino]-6-(2-hydroxyphényl)-5-méthylpyridazine.

1,2 g du produit obtenu précédemment sont mis en solution dans 60 ml d'acide bromhydrique à 48 % et la solution est chauffée à reflux pendant 48 heures. Le mélange réactionnel est concentré sous vide et le résidu est repris dans l'eau saturée de carbonate de potassium. L'huile formée est extraite au dichlorométhane, la phase organique est séparée, lavée avec une solution saturée de chlorure de sodium, séchée avec $MgSO_4$ et concentrée sous vide. Le résidu est trituré dans l'éther et filtré puis purifié par chromatographie sur alumine, éluant : acétate d'éthyle-méthanol 8-2 (v/v) plus 2 % de triéthylamine. La concentration des fractions de produit pur fournit 0,8 g du produit attendu.

F = 208 °C.

En procédant comme il est décrit à l'exemple 1 et en faisant varier la chloro-3 pyridazine de départ, on synthétise les composés répertoriés dans le tableau 1 ci-dessous.

## TABLEAU 1

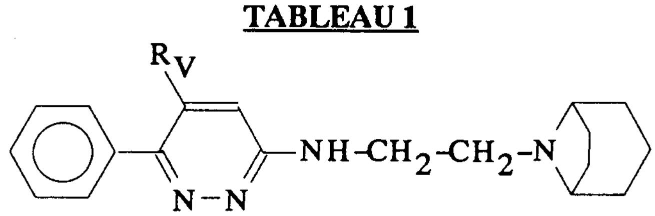

| Exemple n° | $R_V$ | Sel | Point de fusion ; °C |
|---|---|---|---|
| 2 | $-CH_3$ | 2HCl | 226 |
| 3 | $-CH_2CH_2CH_3$ | dioxalate | 150 |

EXEMPLE 4

Difumarate de 3-(8-éthyl-8-azabicyclo[3.2.1]oct-3-ylamino)-6-phényl-5-propylpyridazine.

$R_6$ = H ; $R_V$ = $-CH_2CH_2CH_3$ ; $n_1$ = $n_2$ = 0 ;

T = 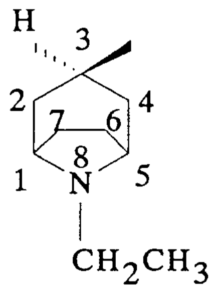

1 g de 3-chloro-6-phényl-5-propylpyridazine et 1 g de diamine (IIa) sont chauffés à 160 °C en autoclave pendant une nuit. Le mélange réactionnel est repris dans le dichlorométhane et lavé avec une solution aqueuse saturée de carbonate de sodium. Les phases organiques sont décantées, séchées sur $MgSO_4$, filtrées et concentrées sous vide. Le résidu est chromatographié sur gel de silice, éluant : dichlorométhane-méthanol 97-3 (v/v). La concentration des fractions pures fournit un résidu qui cristallise avec deux équivalents d'acide fumarique.

m = 0,44g

F = 82 °C.

EXEMPLE 5

Difumarate de 3-(8-éthyl-8-azabicyclo[3.2.1]oct-3-ylamino)-6-phényl-5-propylpyridazine.

$R_6$ = H ; $R_V$ = $-CH_2CH_2CH_3$ ; $n_1$ = $n_2$ = 0 ;

T = 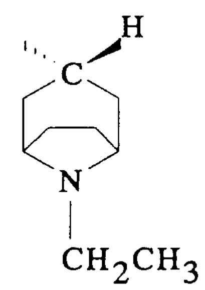

En procédant selon l'exemple 4 et en remplaçant la diamine (IIa) par la diamine (IIe), on prépare le composé ci-dessus.

m = 0,5 g

F = 139 °C.

EXEMPLE 6

Difumarate de 3-(1-azabicyclo[3.3.0]oct-5-yl)méthylamino-5-méthyl-6-phénylpyridazine.

$R_6$ = H ; $R_V$ = $-CH_3$ ; $Y_1$ = H ; $n_1$ = 1 ; $n_2$ = 0 ;

T = 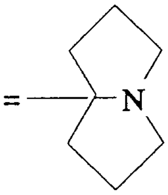

1,52 g de 1-azabicyclo[3.3.0]oct-5-ylméthylamine, 2,21 g de 3-chloro-5-méthyl-6-phénylpyridazine et 0,58 g de chlorure d'ammonium sont mis en solution dans 10 ml de pentanol et chauffés à reflux sous argon pendant 24 heures. Le mélange réactionnel est concentré sous vide en azéotropant avec de l'eau. Le résidu est alcalinisé avec une solution aqueuse de carbonate de potassium à 10 % puis extrait avec de l'acétate d'éthyle. La phase organique est séparée puis on ajoute 30 ml d'une solution aqueuse d'acide citrique à 10 %. La phase aqueuse est séparée, lavée deux fois avec de l'acétate d'éthyle et alcalinisée avec une solution d'hydroxyde de sodium à 33 % jusqu'a pH = 13. L'huile formée est extraite à l'acétate d'éthyle, la phase organique est décantée, séchée sur $Na_2SO_4$, filtrée et chromatographiée sur alumine, éluant : acétate d'éthyle puis acétate d'éthyle-méthanol 9-1 (v/v) additionné de 2 % de triéthylamine. La concentration des fractions de produit pur fournit 1 g du produit attendu.

0,6 g de la base obtenue sont mis en solution dans un minimum d'acétone puis on ajoute 0,45 g d'acide fumarique en solution dans l'acétone.

Le cristaux sont filtrés et on obtient 0,1 g de fumarate.

F = 153,3 °C.

En procédant comme il est décrit à l'exemple 6 et en faisant varier la 3-chloropyridazine de départ, on prépare le composé suivant :

## TABLEAU 2

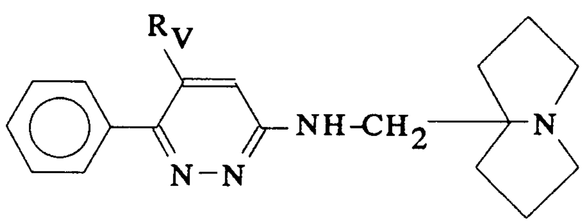

| Exemple n° | $R_V$ | Sel | Point de fusion ; °C |
|---|---|---|---|
| 7 | $-CH_2CH_2CH_3$ | difumarate | 179 |

Les propriétés pharmacologiques des composés selon l'invention ont été étudiées et en particulier leur affinité pour les récepteurs cholinergiques muscariniques de type $M_1$ et $M_2$.

*In vitro*, les composés (I) ont été essayés suivant la technique décrite par L. Potter et al., J. Pharmacol. Exp. Ther., 1989, 284, 974-978 en ce qui concerne leur affinité pour les récepteurs de type $M_1$ et selon la

technique décrite par Hammer R. et al., Life Science, 1986, 38, 1653-1662, en ce qui concerne leur affinité pour les récepteurs de type $M_2$.

Les composés selon l'invention présentent une bonne affinité pour les récepteurs de type $M_1$ et une spécificité marquée pour les récepteurs centraux de type $M_1$ vis-à-vis des récepteurs de type $M_2$.

A titre d'exemple, les composés (I) selon l'invention ont montré une concentration inhibitrice 50 ($CI_{50}$) exprimée en nanomole par litre, de l'ordre de 3,2 et 110 respectivement sur les récepteurs $M_1$ et $M_2$.

*In vivo*, les composés selon l'invention ont été essayés sur le test des rotations induites par la pirenzépine intrastriatale décrit par Worms P. et al., Psychopharmacology, 1987, 93, 489-493.

A la dose de 0,3 mg par kg de poids corporel, per os, les produits selon l'invention inhibent fortement le nombre des rotations induites par la pirenzépine. Ainsi à titre d'exemple, les composés (I) selon l'invention inhibent de 62 % les rotations induites par la pirenzépine.

Par suite les composés (I) peuvent être utilisés en tant que médicaments.

Les composés selon l'invention présentent une bonne affinité pour les récepteurs muscariniques et une bonne activité dans les tests d'amnésie induite par la scopolamine ou la pirenzépine. Ils permettent d'envisager l'utilisation des produits selon l'invention dans tous les cas où se manifeste un déficit cholinergique et notamment pour le traitement des troubles mnésiques cognitifs, des syndromes dégénératifs liés à la sénescence et aux démences séniles.

Enfin les composés selon l'invention n'ont donné aucun signe de toxicité aux doses où ils sont actifs.

Selon un autre de ses aspects la présente demande concerne donc les compositions pharmaceutiquement acceptables contenant au moins un des composés de formule (I) ou un de leurs sels en tant que principe actif.

Dans les compositions pharmaceutiques de la présente invention pour l'administration orale, sublinguale, transdermique ou rectale, les principes actifs de formule (I) ci-dessus penvent être administrés sous forme unitaire d'administration, en mélange avec les supports pharmaceutiques classiques, aux êtres humains notamment pour le traitement des troubles mnésiques cognitifs ou des syndromes dégénératifs. Les formes unitaires d'administration appropriées comprennent les formes par voie orale, telles que les comprimés, les gélules, les poudres, les granules et les solutions ou suspensions orales, les formes d'administration sublinguale et buccale, les formes d'administration sous cutanée, intramusculaire ou intraveineuse et les formes d'administration rectale.

Afin d'obtenir l'effet désiré, la dose de principe actif peut varier entre 0,5 et 500 mg par jour.

Chaque dose unitaire peut contenir de 0,1 à 100 mg d'ingrédient actif en combinaison avec un support pharmaceutique. Cette dose unitaire peut être administrée 1 à 5 fois par jour.

Lorsqu'on prépare une composition solide sous forme de comprimés, on mélange le principe actif avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans les gélules molles ou dures.

Les poudres ou les granulés dispersibles dans l'eau peuvent contenir le principe actif en mélange avec des agents de dispersion ou des agents mouillants, ou des agents de mise en suspension comme la polyvinylpyrrolidone, de même qu'avec des édulcorants ou des correcteurs de goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol et le butylèneglycol.

Le principe actif peut être formulé également sous forme de microcapsules éventuellement avec un ou plusieurs supports ou additifs.

A titre de préparation galénique on peut préparer des gélules contenant :

| | |
|---|---|
| Composé de l'exemple 1 | 0,010 g |
| Lactose | 0,050 g |
| Stéarate de magnésium | 0,005 g. |

**Revendications**

**Revendications pour les Etats contractants suivants : AT, BE, CH, DE, DK, FR, GB, IT, LI, LU, NL, PT, SE**

**1.** Composés de formule :

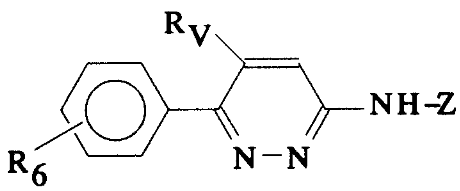

(I)

dans laquelle :

- $R_V$ représente un groupe alkyle droit ou ramifié en $C_1$-$C_4$ ;
- $R_6$ représente l'hydrogène, un alcoxy en $C_1$-$C_3$ ou un groupe hydroxyle;
- Z représente un groupe :

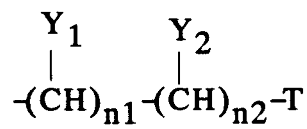

dans lequel :

. $n_1$ et $n_2$ représentent indépendamment zéro ou un,
. $Y_1$ et $Y_2$ représentent chacun l'hydrogène,
. T représente un hétérocycle choisi parmi :

a)

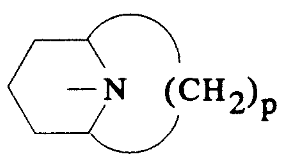

lorsque $n_1 = n_2 = 1$
et p représente alors 2 ou 3;

b)

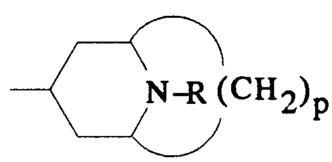

lorsque $n_1 = n_2 = 0$
et p représente alors 2 ou 3, R = alkyle $C_1$-$C_3$ ;

c)

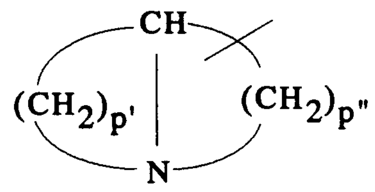

lorsque $n_1 = 1$, $n_2 = 0$
et p' et p" représentent alors 3 ou 4;
ainsi que leurs sels avec des acides organiques ou minéraux.

**2.** Composé selon la revendication 1 caractérisé en ce que Z-NH- est représenté par le groupe 2-(8-azabicyclo[3.2.1]oct-8-yl)éthylamino, ou un de ses sels pharmaceutiquement acceptables.

**3.** Composé selon la revendication 1 caractérisé en ce que Z-NH est représenté par le groupe 8-éthyl-8-azabicyclo[3.2.1]oct-3-ylamino, ou un de ses sels pharmaceutiquement acceptables.

**4.** Composé selon la revendication 1 caractérisé en ce que Z-NH- est représenté par le groupe 1-azabicyclo[3.3.0]oct-5-ylméthylamino, ou un de ses sels pharmaceutiquement acceptables.

**5.** Procédé de préparation d'un composé selon la revendication 1 caractérisé en ce que l'on fait réagir une amine de formule :

$H_2N$-Z (II)

dans laquelle Z est tel que défini dans la revendication 1 sur la 3-chloropyridazine de formule :

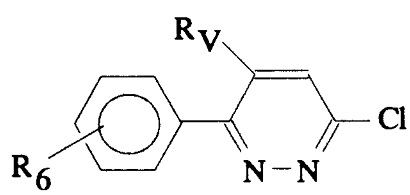

(III)

dans laquelle $R_V$ et $R_6$ sont tels que définis dans la revendication 1 et, éventuellement on salifie le composé ainsi obtenu avec un acide minéral ou organique.

**6.** Composition pharmaceutique contenant, en tant que principe actif, un composé de formule (I) selon l'une quelconque des revendications 1 à 4.

**7.** Composition pharmaceutique selon la revendication 6, sous forme d'unité de dosage, dans laquelle le principe actif est mélangé à au moins un excipient pharmaceutique.

**8.** Composition selon la revendication 7 contenant de 0,5 à 100 mg de principe actif.

**Revendications pour l'Etat contractant suivant : ES**

**1.** Procédé pour l'obtention de composés de formule :

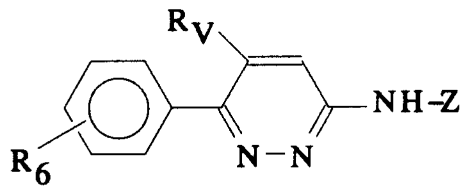

(I)

dans laquelle :

- $R_V$ représente un groupe alkyle droit ou ramifié en $C_1$-$C_4$ ;
- $R_6$ représente l'hydrogène, un alcoxy en $C_1$-$C_3$ ou un groupe hydroxyle;
- Z représente un groupe :

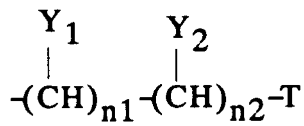

dans lequel :

. $n_1$ et $n_2$ représentent indépendamment zéro ou un,
. $Y_1$ et $Y_2$ représentent chacun l'hydrogène,
. T représente un hétérocycle choisi parmi:

a)

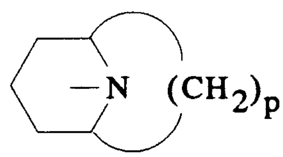

lorsque $n_1 = n_2 = 1$
et p représente alors 2 ou 3 ;

b)

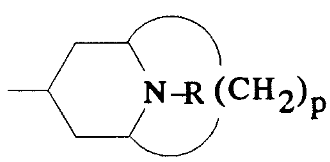

lorsque $n_1 = n_2 = 0$
et p représente alors 2 ou 3, R = alkyle $C_1$-$C_3$ ;

c)

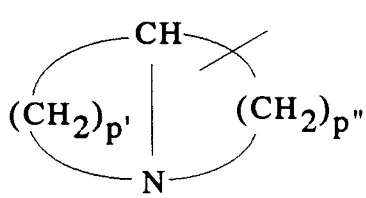

lorsque $n_1 = 1$, $n_2 = 0$
et p' et p'' représentent alors 3 ou 4 ;

ainsi que leurs sels avec des acides organiques ou minéraux, caractérisé en ce que l'on fait réagir une amine de formule :

$H_2N$-Z (II)

dans laquelle Z est tel que défini ci-dessus sur la 3-chloropyridazine de formule :

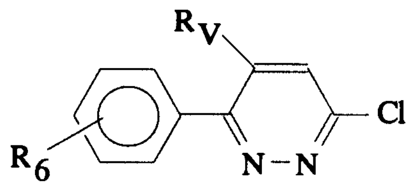

(III)

dans laquelle $R_V$ et $R_6$ sont tels que définis ci-dessus et, éventuellement, on salifie le composé ainsi obtenu avec un acide minéral ou organique.

**2.** Procédé selon la revendication 1, caractérisé en ce que l'on utilise comme amine de formule (II) la 2-(8-azabicyclo[3.2.1]oct-8-yl)éthylamine pour former les composés de formule (I) dans laquelle le groupe -Z-NH- est le groupe 2-(8-azabicyclo[3.2.1]oct-8-yl)éthylamino, ou un de leurs sels pharmaceutiquement acceptables.

**3.** Procédé selon la revendication 1, caractérisé en ce que l'on utilise comme amine de formule (II) la 8-éthyl-8-azabicyclo[3.2.1]oct-3-ylamine, pour former les composés de formule (I) dans laquelle le groupe -Z-NH- est le groupe 8-éthyl-8-azabicyclo[3.2.1]oct-3-ylamino, ou un de leurs sels pharmaceutiquement acceptables.

**4.** Procédé selon la revendication 1, caractérisé en ce que l'on utilise la 1-azabicyclo[3.3.0]oct-5-ylméthylamine comme amine de formule (II), pour former les composés de formule (I) dans laquelle le groupe -Z-NH- est le groupe 1-azabicyclo[3.3.0]oct-5-ylméthylamino, ou un de leurs sels pharmaceutiquement acceptables.

**5.** Procédé pour l'obtention d'une composition pharmaceutique, caractérisé en ce qu'il consiste à mélanger un composé de formule (I) préparé par le procédé selon l'une quelconque des revendications 1 à 4, avec un véhicule pharmaceutiquement acceptable.

**Revendications pour l'Etat contractant suivant : GR**

**1.** Composés de formule :

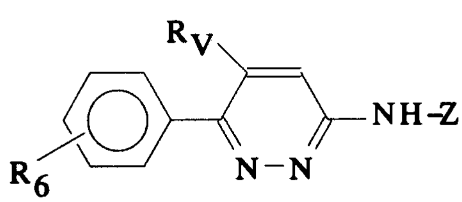

(I)

dans laquelle :
- $R_V$ représente un groupe alkyle droit ou ramifié en $C_1$-$C_4$ ;
- $R_6$ représente l'hydrogène, un alcoxy en $C_1$-$C_3$ ou un groupe hydroxyle;

- Z représente un groupe :

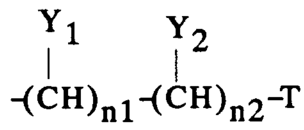

dans lequel :
. $n_1$ et $n_2$ représentent indépendamment zéro ou un,
. $Y_1$ et $Y_2$ représentent chacun l'hydrogène,
. T représente un hétérocycle choisi parmi :
a)

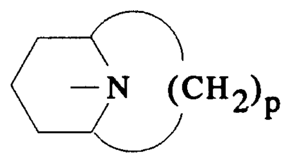

lorsque $n_1 = n_2 = 1$
et p représente alors 2 ou 3;
b)

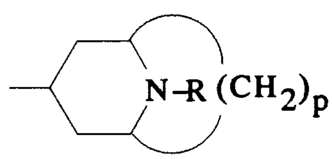

lorsque $n_1 = n_2 = 0$
et p représente alors 2 ou 3, R = alkyle $C_1$-$C_3$ ;
c)

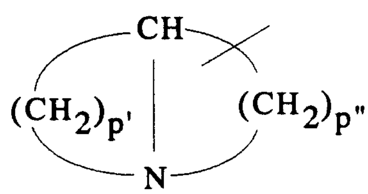

lorsque $n_1 = 1$, $n_2 = 0$
et p' et p'' représentent alors 3 ou 4;
ainsi que leurs sels avec des acides organiques ou minéraux.

**2.** Composé selon la revendication 1 caractérisé en ce que Z-NH- est représenté par le groupe 2-(8-azabicyclo[3.2.1]oct-8-yl)éthylamino, ou un de ses sels pharmaceutiquement acceptables.

**3.** Composé selon la revendication 1 caractérisé en ce que Z-NH est représenté par le groupe 8-éthyl-8-azabicyclo[3.2.1]oct-3-ylamino, ou un de ses sels pharmaceutiquement acceptables.

**4.** Composé selon la revendication 1 caractérisé en ce que Z-NH- est représenté par le groupe 1-azabicyclo[3.3.0]oct-5-ylméthylamino, ou un de ses sels pharmaceutiquement acceptables.

**5.** Procédé de préparation d'un composé selon la revendication 1 caractérisé en ce que l'on fait réagir une amine de formule :

$H_2N$-Z (II)

dans laquelle Z est tel que défini dans la revendication 1 sur la 3-chloropyridazine de formule :

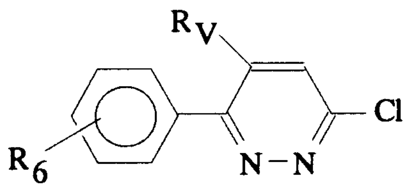

(III)

dans laquelle $R_V$ et $R_6$ sont tels que définis dans la revendication 1 et, éventuellement on salifie le composé ainsi obtenu avec un acide minéral ou organique.

**6.** Procédé pour l'obtention d'une composition pharmaceutique, caractérisé en ce qu'il consiste à mélanger un composé de formule (I) selon l'une quelconque des revendications 1 à 4 avec un véhicule pharmaceutiquement acceptable.

**Claims**
**Claims for the following Contracting States : AT, BE, CH, DE, DK, FR, GB, IT, LI, LU, NL, PT, SE**

**1.** Compounds of the formula:

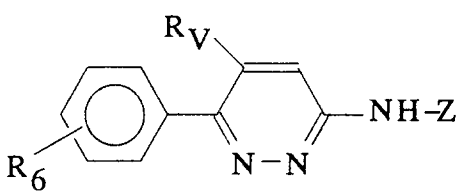

(I)

in which :
- $R_V$ is a linear or branched $C_1$-$C_4$ alkyl group;
- $R_6$ is hydrogen, a $C_1$-$C_3$ alkoxy or a hydroxyl group;
- Z is a group :

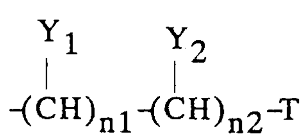

in which :
. $n_1$ and $n_2$ independently are zero or one,
. $Y_1$ and $Y_2$ are each hydrogen,
. T is a heterocycle selected from :
a)

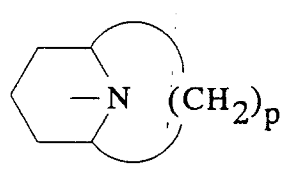

when $n_1 = n_2 = 1$
and p is then 2 ou 3 ;
b)

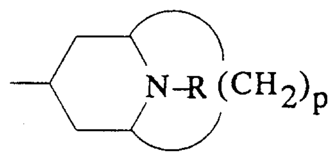

when $n_1 = n_2 = 0$
and p is 2 or 3 and R = $C_1$-$C_3$ alkyl ;
c)

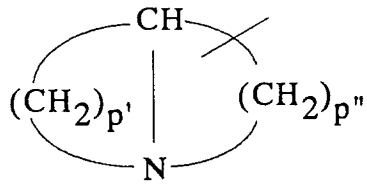

when $n_1 = 1$, $n_2 = 0$
and p' and p'' are 3 or 4;
as well as their salts with organic or mineral acids.

**2.** Compound according to claim 1, characterized in that Z-NH- is represented by the group 2-(8-azabicyclo[3.2.1]oct-8-yl)ethylamino, or one of its pharmaceutically acceptable salts.

**3.** Compound according to claim 1, characterized in that Z-NH- is represented by the group 8-ethyl-8-azabicyclo[3.2.1]oct-3-ylamino, or one of its pharmaceutically acceptable salts

**4.** Compound according to claim 1, characterized in that Z-NH- is represented by the group 1-azabicyclo-[3.3.0]oct-5-ylmethylamino, or one of its pharmaceutically acceptable salts.

**5.** A method of preparing a compound according to claim 1, characterized in that it comprises reacting an amine of the formula :

$H_2N$-Z (II)

in which Z is as defined in claim 1, with the 3-chloropyridazine of the formula

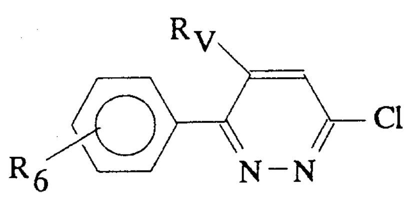

(III)

in which $R_V$ and $R_6$ are as defined in claim 1, and, if desired, converting the resulting compound to a salt with a mineral or organic acid.

**6.** Pharmaceutical composition containing, as the active principle, a compound of formula (I) according to any one of claims 1 to 4.

**7.** Pharmaceutical composition according to claim 6, in the form of a dosage unit, in which the active principle is mixed with at least one pharmaceutical excipient.

**8.** Composition according to claim 7 containing from 0.5 to 100 mg of active principle.

**Claims for the following Contracting State : ES**

**1.** A method of obtaining compounds of the formula :

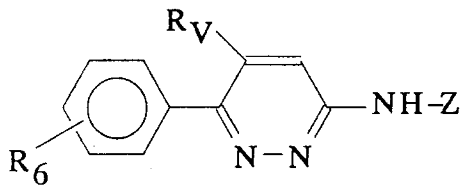

(I)

in which :

- $R_V$ is a linear or branched $C_1$-$C_4$ alkyl group ;
- $R_6$ is hydrogen, a $C_1$-$C_3$ alkoxy or a hydroxyl group ;
- Z is a group :

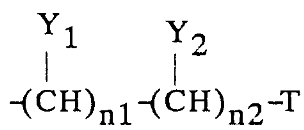

in which :

. $n_1$ and $n_2$ independently are zero or one,
. $Y_1$ and $Y_2$ are each hydrogen,
. T is a heterocycle selected from :
a)

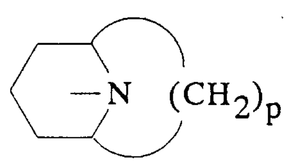

when $n_1 = n_2 = 1$
and p is then 2 ou 3 ;
b)

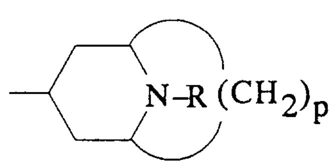

when $n_1 = n_2 = 0$
and p is 2 or 3 and R = $C_1$-$C_3$ alkyl ;

c)

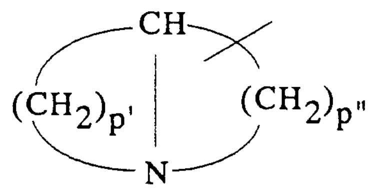

when $n_1 = 1$, $n_2 = 0$
and p' and p'' are 3 or 4;
as well as their salts with organic or mineral acids, characterized in that an amine of the formula :

$H_2N$-Z (II)

in which Z is as defined above, is reacted with the 3-chloropyridazine of the formula :

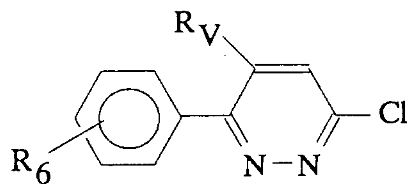

(III)

in which $R_V$ and $R_6$ are as defined above and optionally converting the resulting compound to a salt with a mineral or organic acid.

**2.** A method according to claim 1, characterized in that an amine of the formula (II) is used which is 2-(8-azabicyclo[3.2.1]oct-8-yl)ethylamine to form the compounds of the formula (I) in which the group -Z-NH is the group 2-(8-azabicyclo[3.2.1]oct-8-yl)ethylamino, or one of their pharmaceutically acceptable salts.

**3.** A method according to claim 1, characterized in that an amine of the formula (II) is used which is 8-ethyl-8-azabicyclo[3.2.1]oct-3-ylamine to form the compounds of the formula (I) in which the group -Z-NH is the group 8-ethyl-8-azabicyclo[3.2.1]oct-3-ylamino, or one of their pharmaceutically acceptable salts.

**4.** A method according to claim 1, characterized in that an amine of the formula (II) is used which is 1-azabicyclo[3.3.0]oct-5-ylmethylamino to form the compounds of the formula (I) in which the group -Z-NH is the group 1-azabicyclo[3.3.0]oct-5-ylmethylamino, or one of their pharmaceutically acceptable salts.

**5.** A method of obtaining a pharmaceutical composition, characterized in that it consists in mixing a compound of formula (I), prepared by the method according to any one of claims 1-4, with a pharmaceutically acceptable carrier.

**Claims for the following Contracting State : GR**

**1.** Compounds of the formula:

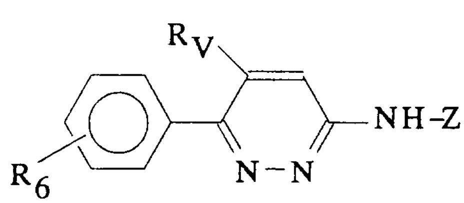

(I)

in which:

- $R_V$ is a linear or branched $C_1$-$C_4$ alkyl group;
- $R_6$ is hydrogen, a $C_1$-$C_3$ alkoxy or a hydroxyl group ;
- Z is a group :

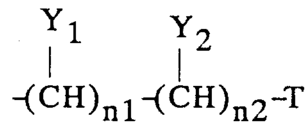

in which :

. $n_1$ and $n_2$ independently are zero or one,
. $Y_1$ and $Y_2$ are each hydrogen,
. T is a heterocycle selected from :

a)

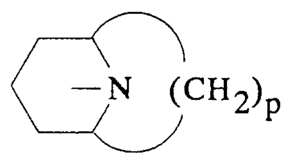

when $n_1 = n_2 = 1$
and p is then 2 ou 3;

b)

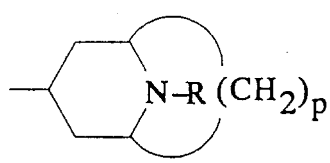

when $n_1 = n_2 = 0$
and p is 2 or 3 and R = $C_1$-$C_3$ alkyl ;

c)

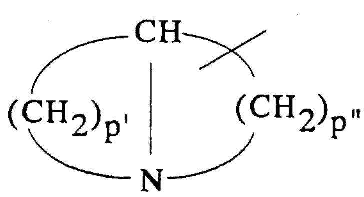

when $n_1 = 1$, $n_2 = 0$
and p' and p'' are 3 or 4 ;

as well as their salts with organic or mineral acids.

**2.** Compound according to claim 1, characterized in that Z-NH- is represented by the group 2-(8-azabicyclo[3.2.1]oct-8-yl)ethylamino, or one of its pharmaceutically acceptable salts.

**3.** Compound according to claim 1, characterized in that Z-NH- is represented by the group 8-ethyl-8-azabicyclo[3.2.1]oct-3-ylamino, or one of its pharmaceutically acceptable salts.

4. Compound according to claim 1, characterized in that Z-NH- is represented by the group 1-azabicyclo-[3.3.0]oct-5-ylmethylamino, or one of its pharmaceutically acceptable salts.

5. A method of preparing a compound according to claim 1, characterized in that it comprises reacting an amine of the formula :

$H_2N\text{-}Z$ (II)

in which Z is as defined in claim 1, with the 3-chloropyridazine of the formula

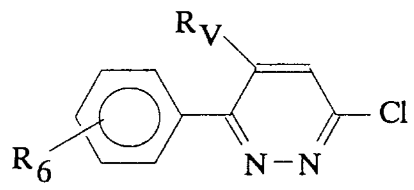

(III)

in which $R_V$ and $R_6$ are as defined in claim 1, and, if desired, converting the resulting compound to a salt with a mineral or organic acid.

6. A method for obtaining a pharmaceutical composition, characterized in that it consists in mixing a compound of formula (I) according to any one of claims 1-4 with a pharmaceutically acceptable carrier.

**Patentansprüche**
**Patentansprüche für folgende Vertragsstaaten : AT, BE, CH, DE, DK, FR, GB, IT, LI, LU, NL, PT, SE**

1. Verbindungen der Formel

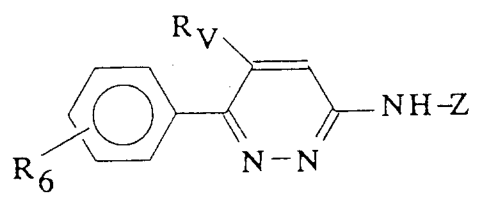

(I)

worin
- $R_V$ eine gerade oder verzweigte $C_1$-$C_4$-Alkylgruppe darstellt;
- $R_6$ Wasserstoff, $C_1$-$C_3$-Alkoxy oder eine Hydroxylgruppe darstellt,
- Z eine Gruppe

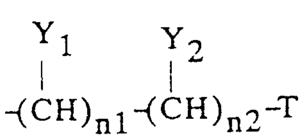

darstellt,
worin
. $n_1$ und $n_2$ unabhängig voneinander Null oder 1 darstellen,
. $Y_1$ und $Y_2$ jeweils Wasserstoff darstellen,
. T einen Heterozyklus darstellt, ausgewählt aus

a)

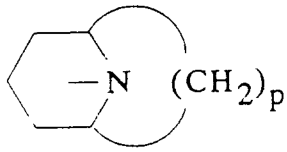

wenn $n_1 = n_2 = 1$
und p dann für 2 oder 3 steht;
b)

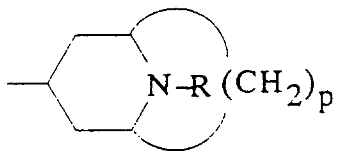

wenn $n_1 = n_2 = 0$
und p dann für 2 oder 3 steht, R = $C_1$-$C_3$-Alkyl;
c)

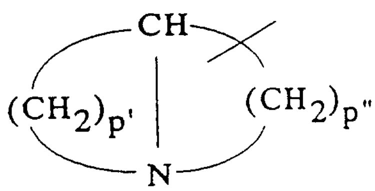

wenn $n_1 = 1$, $n_2 = 0$
und p' und p'' dann für 3 oder 4 stehen;
sowie die Salze derselben mit organischen oder Mineralsäuren.

2. Verbindung nach Anspruch 1, dadurch gekennzeichnet, daß Z-NH-die Gruppe 2-(8-Azabicyclo[3.2.1]oct-8-yl)-ethylamino darstellt, oder ein pharmazeutisch akzeptables Salz derselben.

3. Verbindung nach Anspruch 1, dadurch gekennzeichnet, daß Z-NH die Gruppe 8-Ethyl-8-azablcyclo-[3.2.1]oct-3-ylamino darstellt, oder ein pharmazeutisch akzeptables Salz derselben.

4. Verbindung nach Anspruch 1, dadurch gekennzeichnet, daß Z-NH-die Gruppe 1-Azabicyclo[3.3.0]oct-5-ylmethylamino darstellt, oder ein pharmazeutisch akzeptables Salz derselben.

5. Verfahren zur Herstellung einer Verbindung nach Anspruch 1, dadurch gekennzeichnet, daß ein Amin der Formel

$H_2N$-Z (II),

worin Z wie in Anspruch 1 definiert ist, mit 3-Chlorpyridazin der Formel

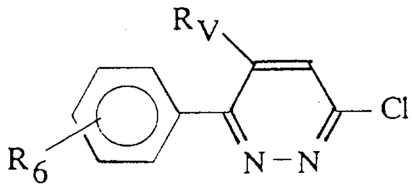

(III)

,

worin $R_V$ und $R_6$ wie in Anspruch 1 definiert sind, zur Umsetzung gebracht und gegebenenfalls die so erhaltene Verbindung mit einer Mineral- oder organischen Säure in das Salz übergeführt wird.

6. Pharmazeutische Zusammensetzung, die als Wirkstoff eine Verbindung der Formel (I) nach einem der Ansprüche 1 bis 4 enthält.

7. Pharmazeutische Zusammensetzung nach Anspruch 6 in Dosiseinheitsform, worin der Wirkstoff mit mindestens einem pharmazeutischen Exzipienten gemischt ist.

8. Zusammensetzung nach Anspruch 7, die 0,5 bis 100 mg Wirkstoff enthält.

**Patentansprüche für folgenden Vertragsstaat : ES**

1. Verfahren zur Herstellung von Verbindungen der Formel

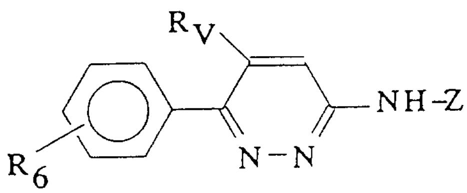

(I)

worin
- $R_V$ eine gerade oder verzweigte $C_1$-$C_4$-Alkylgruppe darstellt;
- $R_6$ Wasserstoff, $C_1$-$C_3$-Alkoxy oder eine Hydroxylgruppe darstellt,
- Z eine Gruppe

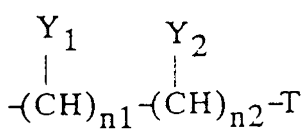

darstellt,
worin
. $n_1$ und $n_2$ unabhängig voneinander Null oder 1 darstellen,
. $Y_1$ und $Y_2$ jeweils Wasserstoff darstellen,
. T einen Heterozyklus darstellt, ausgewählt aus
a)

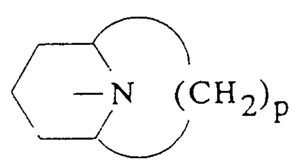

wenn $n_1 = n_2 = 1$
und p dann für 2 oder 3 steht;
b)

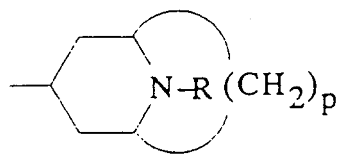

wenn $n_1 = n_2 = 0$
und p dann für 2 oder 3 steht, R = $C_1$-$C_3$-Alkyl;
c)

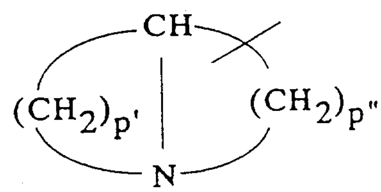

wenn $n_1 = 1$, $n_2 = 0$
und p' und p'' dann für 3 oder 4 stehen;

sowie von deren Salzen mit organischen oder Mineralsäuren, dadurch gekennzeichnet, daß ein Amin der Formel

$H_2N$-Z (II),

worin Z wie oben definiert ist, mit 3-Chlorpyridazin der Formel

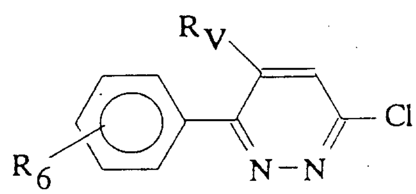

(III)

worin $R_V$ und $R_6$ wie oben definiert sind, zur Umsetzung gebracht und gegebenenfalls die so erhaltene Verbindung mit einer Mineral- oder organischen Säure in das Salz übergeführt wird.

2. Verfahren nach Anspruch 1, dadurch gekennzeichnet, daß als Amin der Formel (II) 2-(8-Azabicyclo-[3.2.1]oct-8-yl)-ethylamin zur Bildung von Verbindungen der Formel (I), worin die Gruppe -Z-NH- die Gruppe 2-(8-Azabicyclo[3.2.1]oct-8-yl)-ethylamino ist, oder eines ihrer pharmazeutisch akzeptabler Salze verwendet wird.

3. Verfahren nach Anspruch 1, dadurch gekennzeichnet, daß als Amin der Formel (II) 8-Ethyl-8-azabicyclo[3.2.1]oct-3-ylamin zur Bildung von Verbindungen der Formel (I), worin die Gruppe Z-NH die Gruppe 8-Ethyl-8-azabicyclo[3.2.1]oct-3-ylamino ist, oder eines ihrer pharmazeutisch akzeptabler Salze verwendet wird.

4. Verfahren nach Anspruch 1, dadurch gekennzeichnet, daß als Amin der Formel (II) 1-Azabicyclo[3.3.0]-oct-5-ylmethylamin zur Bildung von Verbindungen der Formel (I), worin die Gruppe Z-NH die Gruppe 1-Azabicyclo[3.3.0]oct-5-ylmethylamino ist, oder eines ihrer pharmazeutisch akzeptabler Salze verwendet wird.

5. Verfahren zur Herstellung einer pharmazeutischen Zusammensetzung, dadurch gekennzeichnet, daß es darin besteht, daß eine Verbindung der Formel (I), hergestellt durch das Verfahren nach einem der Ansprüche 1 bis 4, mit einem pharmazeutisch akzeptablen Träger vermischt wird.

**Patentansprüche für folgenden Vertragsstaat : GR**

1. Verbindungen der Formel

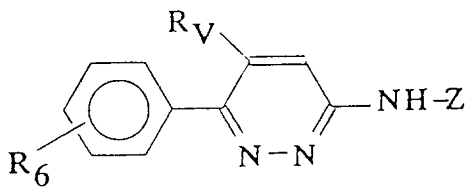

(I)

worin
- $R_V$ eine gerade oder verzweigte $C_1$-$C_4$-Alkylgruppe darstellt;
- $R_6$ Wasserstoff, $C_1$-$C_3$-Alkoxy oder eine Hydroxylgruppe darstellt,
- Z eine Gruppe

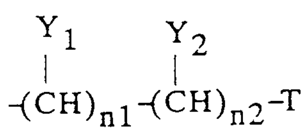

darstellt,
worin
. $n_1$ und $n_2$ unabhängig voneinander Null oder 1 darstellen,
. $Y_1$ und $Y_2$ jeweils Wasserstoff darstellen,
. T einen Heterozyklus darstellt, ausgewählt aus
a)

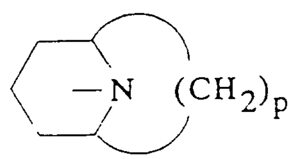

wenn $n_1 = n_2 = 1$
und p dann für 2 oder 3 steht;
b)

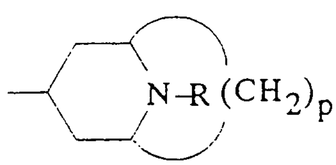

wenn $n_1 = n_2 = 0$
und p dann für 2 oder 3 steht, R = $C_1$-$C_3$-Alkyl;

c)

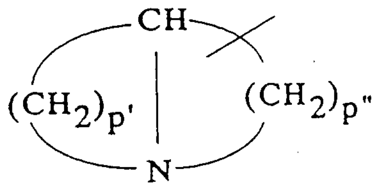

wenn $n_1 = 1$, $n_2 = 0$
und p' und p'' dann für 3 oder 4 stehen;
sowie die Salze derselben mit organischen oder Mineralsäuren.

2. Verbindung nach Anspruch 1, dadurch gekennzeichnet, daß Z-NH die Gruppe 2-(8-Azabicyclo[3.2.1]oct-8-yl)-ethylamino darstellt, oder ein pharmazeutisch akzeptables Salz derselben.

3. Verbindung nach Anspruch 1, dadurch gekennzeichnet, daß Z-NH die Gruppe 8-Ethyl-8-azabicyclo-[3.2.1]oct-3-ylamino darstellt, oder ein pharmazeutisch akzeptables Salz derselben.

4. Verbindung nach Anspruch 1, dadurch gekennzeichnet, daß Z-NH die Gruppe 1-Azabicyclo[3.3.0]oct-5-ylmethylamino darstellt, oder ein pharmazeutisch akzeptables Salz derselben

5. Verfahren zur Herstellung einer Verbindung nach Anspruch 1, dadurch gekennzeichnet, daß ein Amin der Formel

$H_2N$-Z (II),

worin Z wie in Anspruch 1 definiert ist, mit 3-Chlorpyridazin der Formel

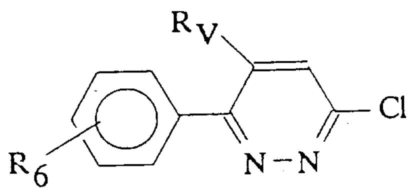

(III) ,

worin $R_V$ und $R_6$ wie in Anspruch 1 definiert sind, zur Umsetzung gebracht und gegebenenfalls die so erhaltene Verbindung mit einer Mineral- oder organischen Säure in das Salz übergeführt wird.

6. Verfahren zur Herstellung einer pharmazeutischen Zusammensetzung, dadurch gekennzeichnet, daß es darin besteht, daß eine Verbindung der Formel (I) nach einem der Ansprüche 1 bis 4 mit einem pharmazeutisch akzeptablen Träger vermischt wird.